# EUROPEAN PATENT APPLICATION

(11) **EP 4 792 839 A1**
(43) Date of publication of application: **19.08.2026**
(21) Application number: 25158091.6
(22) Date of filing: 14.02.2025
(51) Int. Cl.: A61L 24/00

(54) **FLEXIBLE AND DEGRADABLE COMPOSITES FOR BONE FIXATION**

(71) Applicant: Biomedical Bonding AB, 114 28 Stockholm (SE)
(72) Inventor: MALKOCH, Michael, 187 63 TÄBY (SE); SAN JACINTO GARCÍA, Jorge, 113 46 STOCKHOLM (SE); HUTCHINSON, Daniel, 183 78 TÄBY (SE)
(74) Representative: Brann AB

(57) **Abstract**

The present invention relates to a composite material comprising a polymeric network and a ceramic component, wherein the polymeric network is obtained from 1,3,5-Triallyl-1,3,5-triazine-2,4,6(1H,3H,5H)-trione (TATATO) and a mixture of 1,3,5-triazine-2,4,6-trione, 1,3,5-tris(mercaptopropyl) (TMTATO) and tris[(3-mercaptopropionyloxy)-ethyl]-isocyanurate (TEMPIC).

## Description

### FIELD OF INVENTION

The present invention relates to a composite material comprising a polymeric network and a ceramic composite. Said composite material is suitable to be used for bone repair and bone fixation since it has sufficient mechanical properties and is degradable. The invention further relates to a composition used to prepare the composite material.

### BACKGROUND

Bone is a highly dynamic and diverse living tissue. In our bodies, bone tissue exists in compact (cortical bone) or trabecular (cancellous bone) arrangements, varying in mechanical strength and modulus. While cortical bone presents a compressive strength of 130-225 MPa and a Young's modulus of 17-20 GPa, trabecular bone has a modulus of 50-100 MPa and a strength of 5-10 MPa. The composition of the bone extracellular matrix (ECM) is formed by a non-mineralized organic component (collagen) and a mineralized inorganic component (apatite mineralites) comprising 30 and 60 wt % of the composition, respectively. These two components are organized as a nano-composite, where the hydroxyapatite (HA) crystals are embedded into the collagen fibers increasing its compressive strength and toughness.

Despite its high strength, bone is susceptible to fractures caused by traumas; the risk of which increases in elderly people, which are susceptible to develop osteoporosis. In younger people, there are also many factors that increase the risk of suffering fractures such as cancer, obesity and sedentary lifestyles. To treat these fractures, metal plates and screws are widely used clinically, as they provide strong stability to the healing bone. However, they also present some disadvantages. Metal implants are not biodegradable, which may necessitate a second surgery to be removed after bone healing, increasing not only the recovery time of the patient, but also the possibility of infections and complications. Moreover, as these implants are usually made of stainless steel, titanium or its alloys, there is a mismatch in the mechanical properties of metal implants and human bone. This mismatch can result in stress shielding which can promote bone resorption and implant loosening. Due to these disadvantages, alternatives to metal plates such as degradable alloys and composites are attracting attention due to their high versatility. Some of these materials can be tailored to mimic bone composition and/or mechanical properties, increasing their compatibility and reducing the risks of stress shielding between the human bone and the fracture fixator used.

Several synthetic and natural polymers have also been identified as biomaterials for bone tissue engineering. These polymers are usually mixed with ceramics, compensating the low compressive modulus of the polymers and the brittleness of ceramics at the same time. The most common polymers and copolymers used are polyesters such as poly(lactic-co-glycolic) acid (PLGA), polylactic acid (PLA) and polycaprolactone (PCL). Co-polymer systems of PLGA-PCL, PLGA-PLL and PLA-PCL have also been produced, allowing for the tuning of the degradation of the organic matrix. The combination of PLA and PLGA with HA have generated interesting composites that promote remineralization *in vitro* and bone formation *in vivo.* It was also demonstrated that poly(L-lactic acid) (PLLA)/HA composite scaffolds increased the osteoconductive properties and promoted osteoblastic proliferation as well as osteocalcin expression. The most used natural polymers are gelatin, chitosan and collagen, which are used to form composites able to stimulate the formation and deposition of calcium phosphate from simulated body fluid (SBF). However, there are not many polymer-infused composites for bone fixation, apart from acrylate-based ones, which follow a chain growth polymerization mechanism that could lead the leach out of unreacted monomers, damaging the surrounding tissue.

### SUMMARY OF INVENTION

In order to overcome the drawbacks of the prior art the present inventors have developed mechanically strong composites that cure into customizable bone fracture fixators and where the composites are degradable.

A further advantage of the present invention is that the mechanical properties and the degradation time can be tuned through varying the ratio between the monomers and the amount of the filler. Also, despite the degradation of the composite, the mechanical integrity remains during the healing of the tissue,
In a first aspect the present invention relates to a composite material as defined in claim 1.

In a second aspect the present invention relates to a composition comprising monomers, a ceramic component and a initiator, wherein the monomers are TATATO, and a mixture of 1,3,5-triazine-2,4,6-trione, 1,3,5-tris(mercaptopropyl) (TMTATO) and tris[(3-mercaptopropionyloxy)-ethyl]-isocyanurate (TEMPIC) in a 90-70:10-30 molar ratio, and wherein the ceramic component is a mixture of hydroxyapatite, and bio glass or tricalcium phosphate.

### ITEMIZED EMBODIMENTS

Here are non-limiting embodiments of the present invention.

In one embodiment according to any of the aspects of the present invention the mixture of TMTATO and TEMPIC has a molar ratio TMTATO:TEMPIC of 90-50:10-50, preferably 80-60:20-40.

In another embodiment the mixture of TMTATO and TEMPIC is present in an amount of 25-45wt% of the total weight of the composite material, preferably 30-40wt%.

In another embodiment the mixture of TMTATO and TEMPIC has a molar ratio of 90-50:10-50, preferably 80-60:20-40.

In another embodiment the mixture of TMTATO and TEMPIC is present in an amount of 25-45wt% of the total weight of the composite material, preferably 30-40wt%.

In another embodiment the TATATO is present in an amount of 15-30wt% of the total weight of the composite material, preferably 17-25wt%.

In another embodiment the ceramic component is present in an amount of 30-60wt% of the total weight of the composite material, preferably 40-55wt%, more preferably 45-53wt%.

In another embodiment the ceramic component comprises hydroxyapatite and, bio glass and/or tricalcium phosphate, and wherein hydroxyapatite and bio glass and/or tricalcium phosphate is present in an amount of 20-50wt% and 5-30wt% respectively based on the total weight of the composition, preferably 30-45wt% and 7-15wt% respectively.

In one embodiment of the present invention wherein the composite material or the composition comprises an amine catalyst, preferably an unsaturated amine catalyst.

In another embodiment of any of the aspects the mixture of TMTATO and TEMPIC has a molar ratio TMTATO:TEMPIC of 90-50:10-50 and wherein the ceramic component comprises hydroxyapatite and, bio glass and/or tricalcium phosphate, and wherein hydroxyapatite and, bio glass and/or tricalcium phosphate is present in an amount of 20-50wt% and 5-30wt% respectively based on the total weight of the composition.

In another embodiment of the second aspect the mixture of TMTATO and TEMPIC is present in an amount of 25-45wt% of the total weight of the composition, preferably 30-40wt%.

In another embodiment of the second aspect the TATATO is present in an amount of 15-30wt% of the total weight of the composition material, preferably 17-25wt%.

In another embodiment of the second aspect the ceramic component is present in an amount of 30-60wt% of the total weight of the composition, preferably 40-55wt%, more preferably 45-53wt%.

In another embodiment of the second aspect the ceramic component comprises hydroxyapatite and bio glass and/or tricalcium phosphate, and wherein hydroxyapatite and bio glass and/or tricalcium phosphate is present in an amount of 20-50wt% and 5-30wt% respectively based on the total weight of the composition, preferably 30-45wt% and 7-15wt% respectively.

In one embodiment of the second aspect the initiator is a photoinitiator.

In one embodiment of the second aspect of the present invention the initiator is selected from lithium phenyl(2,4,6-trimethylbenzoyl) phosphinate, lithium phenyl-2,4,6-trimethylbenzoylphosphinate, Ommirad 1000 (80% (2-hydroxy-2-methyl-1-phenylpropanone), 20% (1-hydroxycyclohexylphenyl ketone)), Omnirad 4265, Lucirin (main component ethyl(2,4,6-trimethylbenzoyl)-phenyl phosphinate) and Omnirad 4265 (50% Lucirin and 50% Omnirad 481 (1 (1-hydroxycyclohexylphenyl ketone)).

In another embodiment of the second aspect the composition is injectable.

In another embodiment of the first aspect the amount of the polymeric network and the ceramic component constitutes at least 90wt% of the total composite material, preferably at least 95wt%.

In another embodiment of the second aspect the amount of the monomers and the ceramic component constitutes at least 90wt% of the total composition, preferably at least 95wt%.

In yet another embodiment the composite or the composition is suitable for treating hard tissue.

All embodiments may be combined and are applicable unless stated otherwise.

### BRIEF DESCRIPTION OF FIGURES

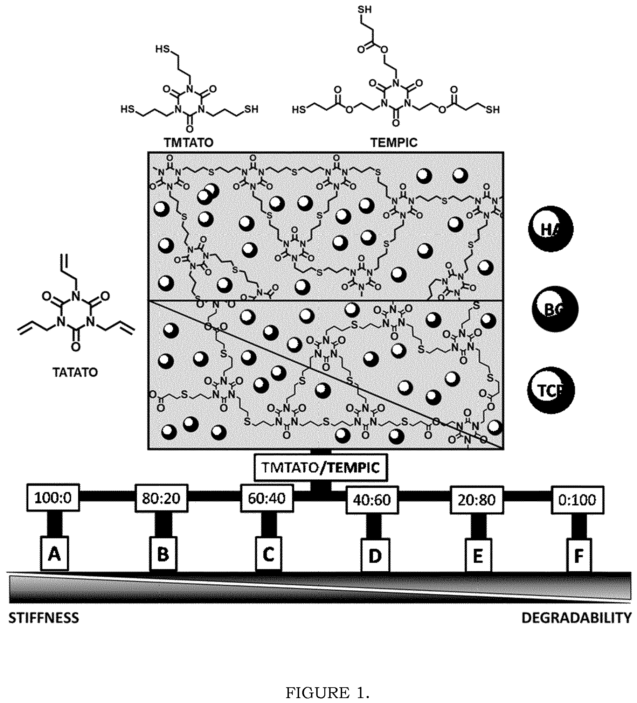

Figure 1, a schematic representation of how the TMTATO and TEMPIC thiol monomers and HA, BG and TCP fillers were mixed to create a library of composites. Formulations A-F show the molar ratio of TMTATO and TEMPIC used.

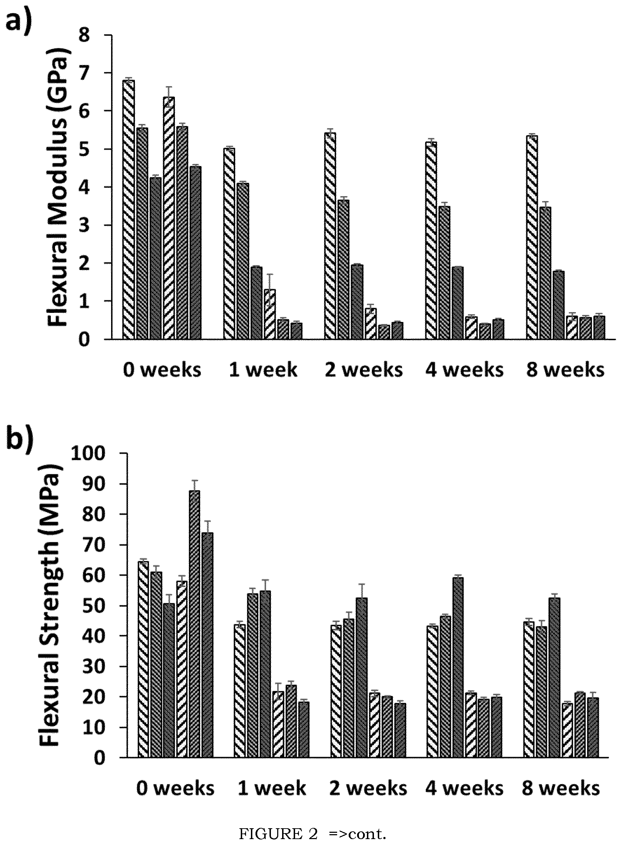

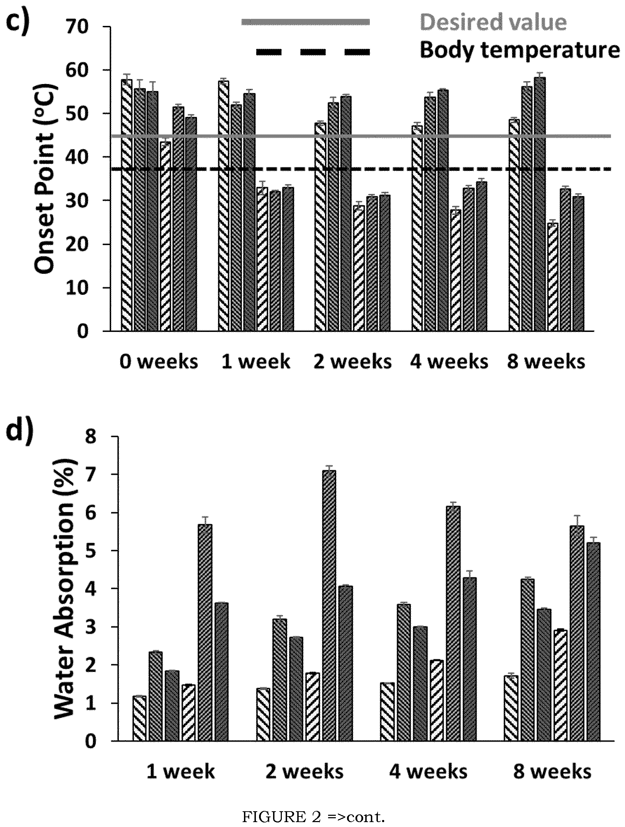

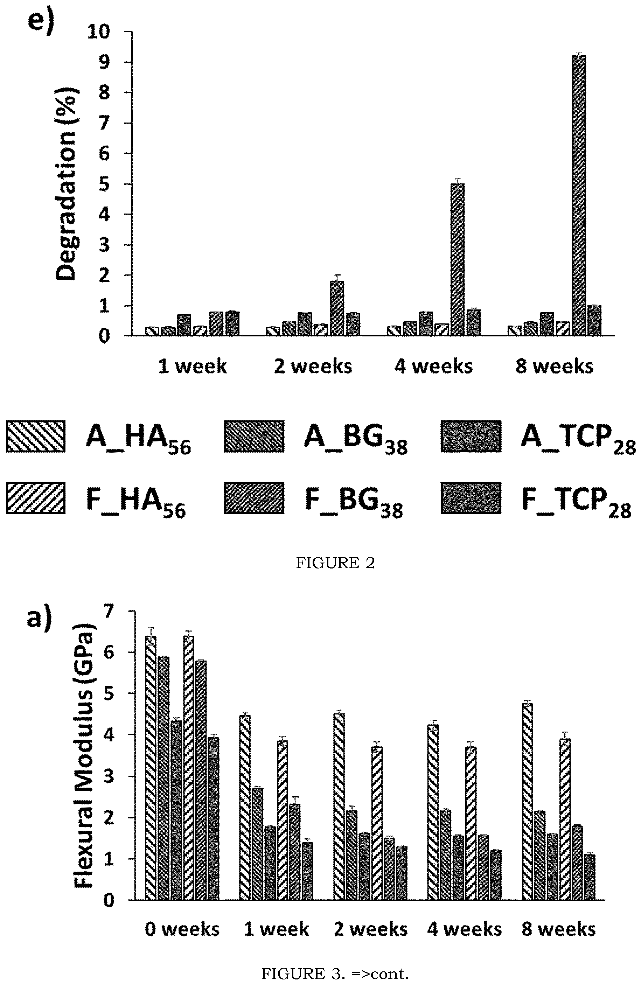

Figure 2, mechanical and degradation properties of composites made with either TMTATO (A) or TEMPIC (F) and either HA, BG or TCP as the reinforcing filler under dry conditions (time = 0 weeks) and at different time points soaked in PBS (pH: 7.4) 37 °C (time = 1-8 weeks): a) flexural modulus, b) flexural strength, c) onset point, d) water absorption and e) degradation as % of mass lost. Mean values shown with standard errors of mean (n=5).

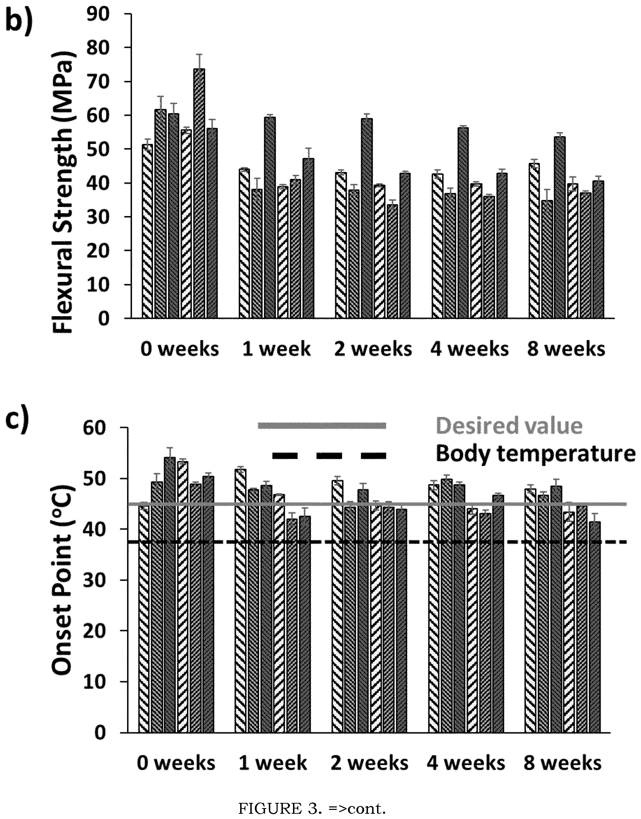

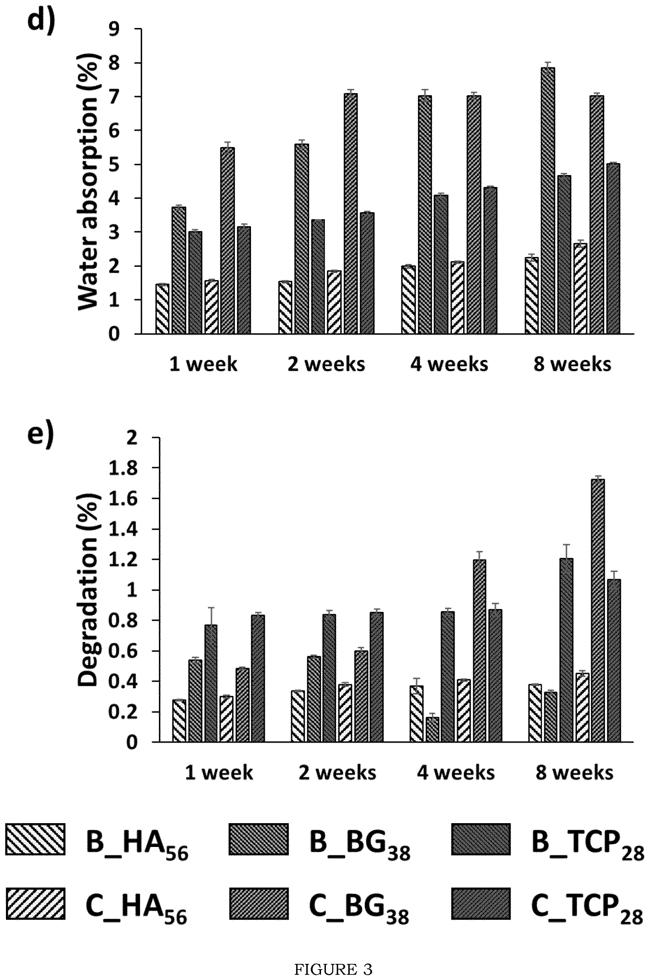

Figure 3, mechanical and degradation properties of composites made with TMTATO and TEMPIC in molar ratios of 80:20 (B) and 60:40 (C) and either HA, BG or TCP as reinforcing fillers under dry conditions (time = 0 weeks) and at different time points soaked in PBS (pH: 7.4) 37 _{°}C (time = 1-8 weeks): a) flexural modulus, b) flexural strength, c) onset point, d) water absorption and e) degradation as % of mass lost. Mean values shown with standard errors of mean (n=5).

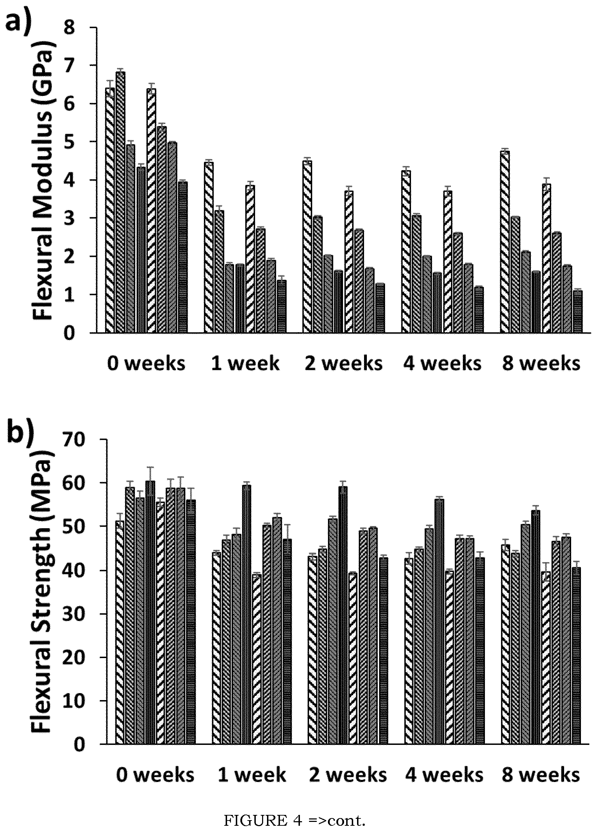

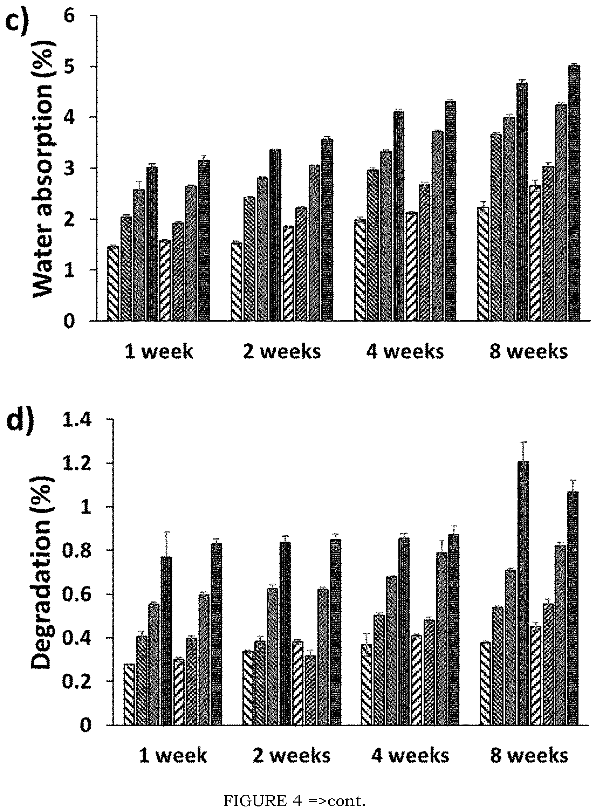

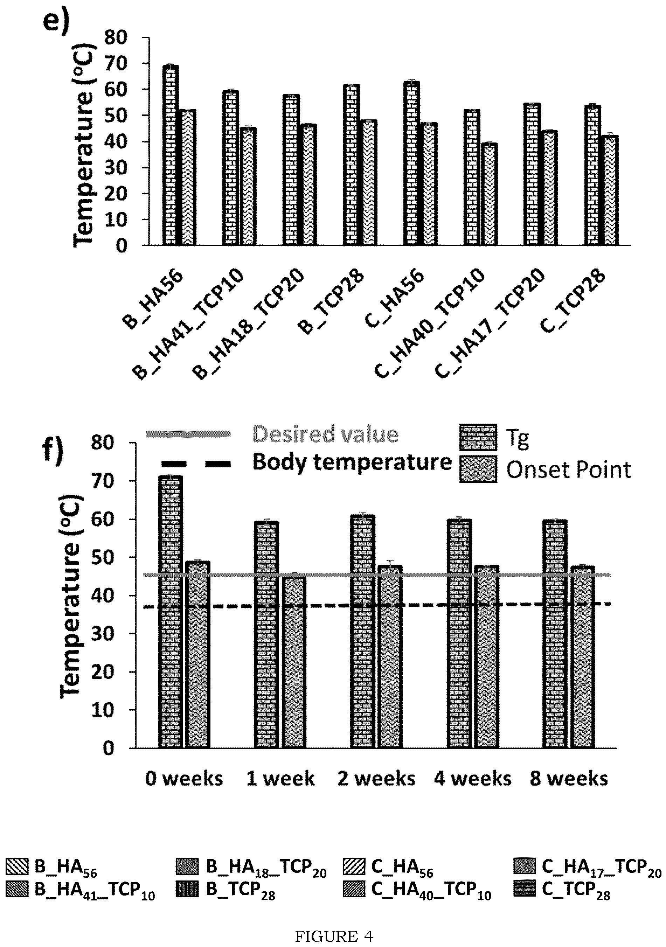

Figure 4, mechanical and degradation properties of composites made with TMTATO and TEMPIC in molar ratios of 80:20 (B) and 60:40 (C) and combinations of HA and TCP as reinforcing agents under dry conditions (time = 0 weeks) and at different time points soaked in PBS (pH: 7.4) 37_{°}C (time = 1-8 weeks): a) flexural modulus, b) flexural strength, c) water absorption, d) degradation as % of mass lost, e) onset point after one week soaked in PBS, and f) onset point of B_HA₄₁_TCP₁₀ at different time points soaking in PBS (0-8 weeks). Mean values shown with standard errors of mean (n=5).

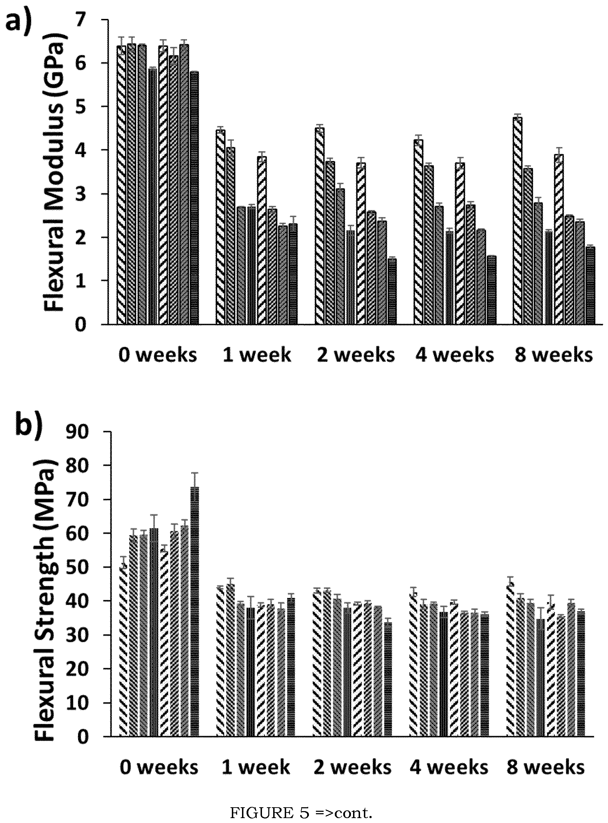

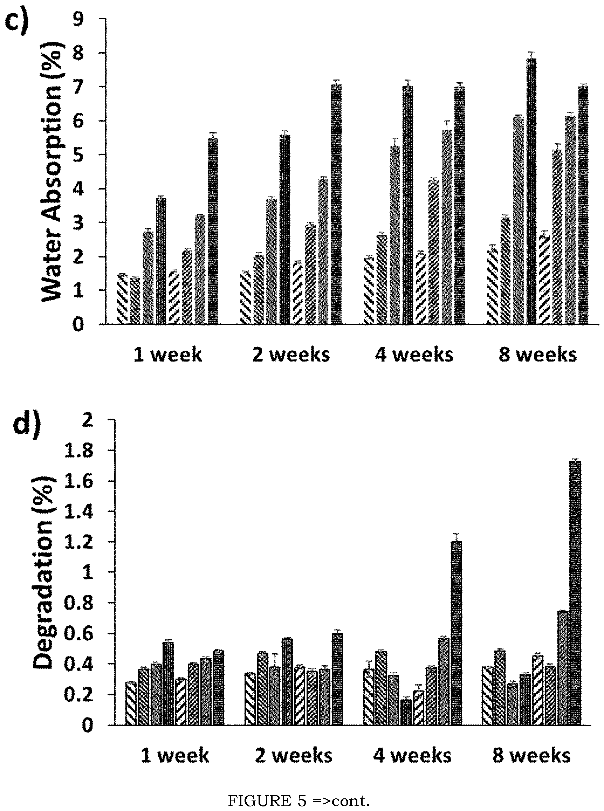

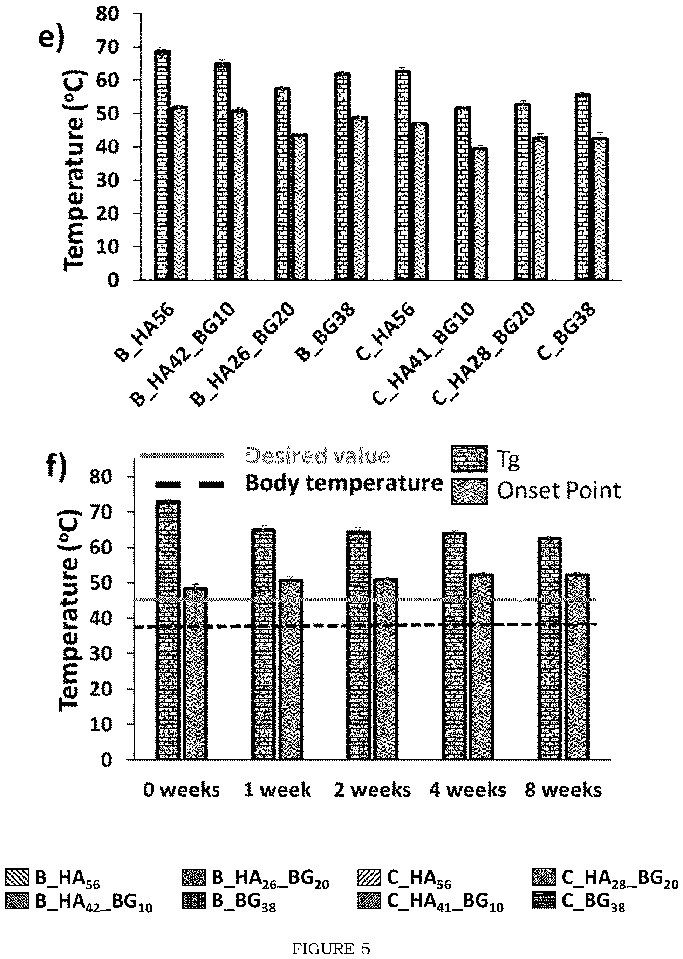

Figure 5, mechanical and degradation properties of composites made with TMTATO and TEMPIC in molar ratios of 80:20 (B) and 60:40 (C) and combinations of HA and BG as reinforcing agents under dry conditions (time = 0 weeks) and at different time points soaked in PBS (pH: 7.4) 37_{°}C (time = 1-8 weeks): a) flexural modulus, b) flexural strength, c) water absorption, d) degradation as % of mass lost e) onset point after one week soaked in PBS (pH= 7.4) at 37-_{°}C, and f) onset point of B_HA_{42_}BG₁₀ at different time points soaking in PBS (0-8 weeks). Mean values shown with standard errors of mean (n=5).

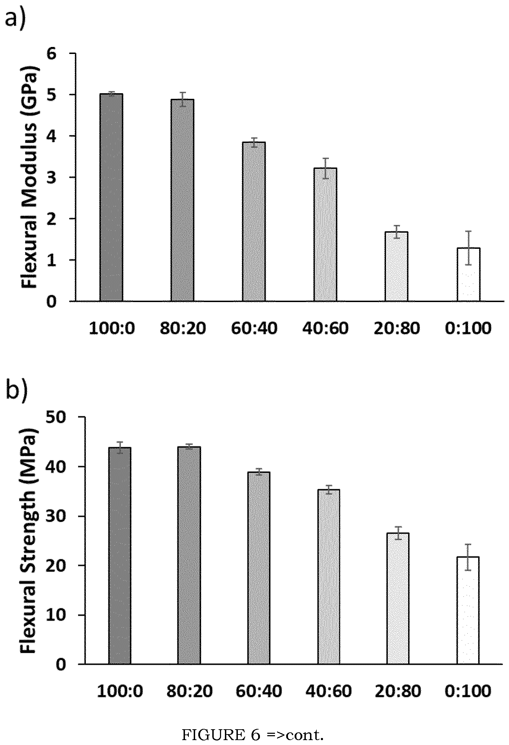

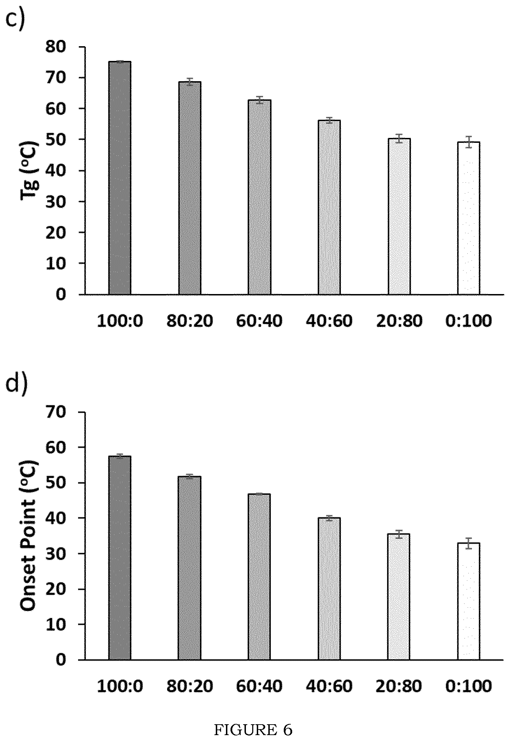

Figure 6, a) Flexural modulus, b) flexural strength, c) Tg and d) onset point of the different TMTATO/TEMPIC ratios after one week soaked in PBS (pH= 7.4) at 37 °C. Mean values shown with standard errors of mean (n=5).

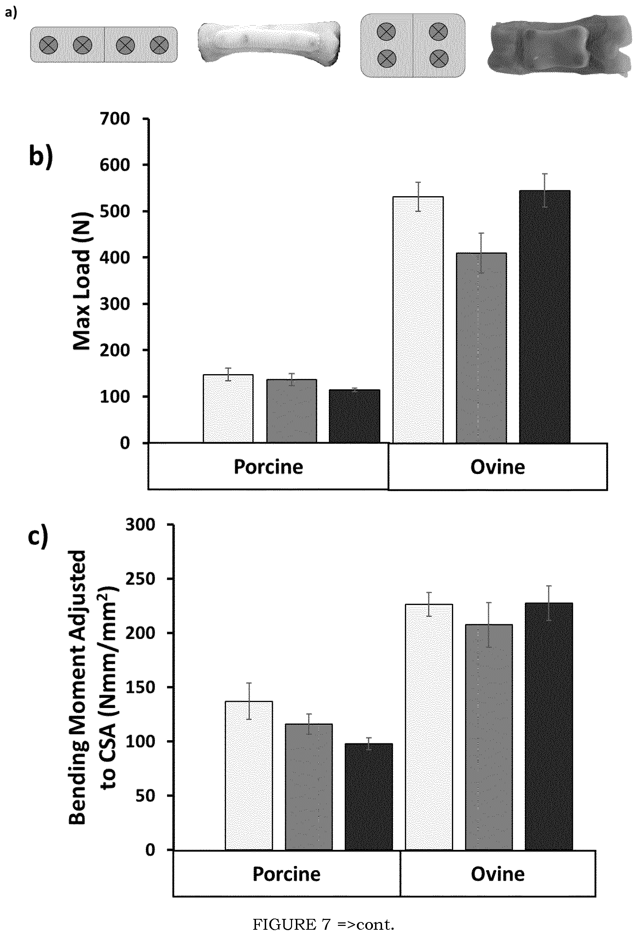

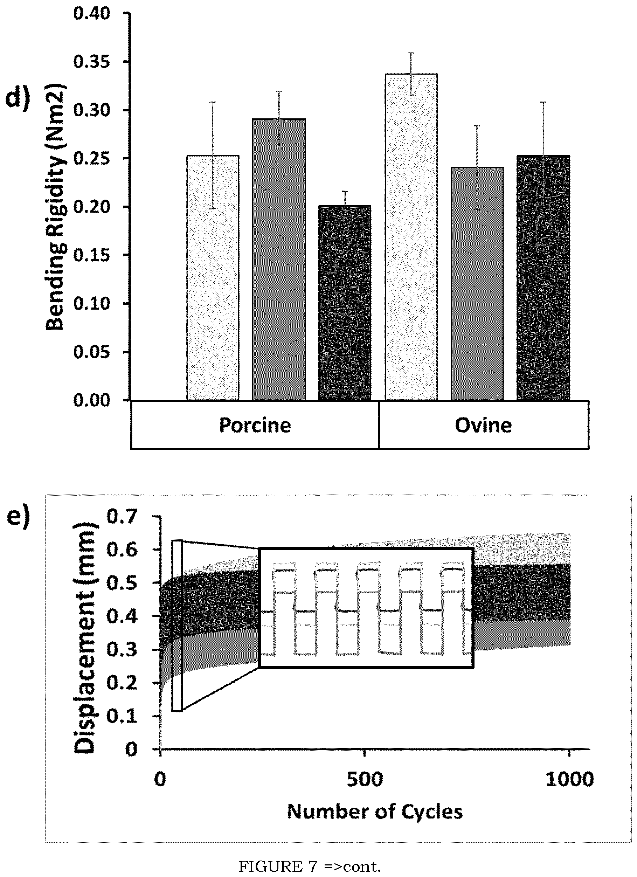

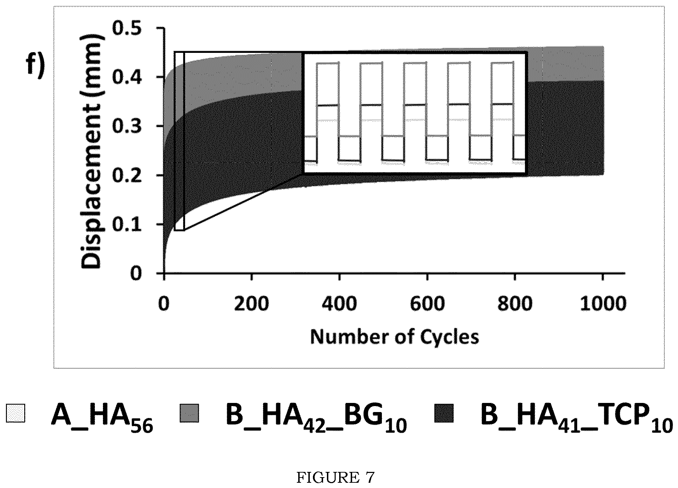

Figure 7, a) configuration of composite and screws used for porcine bones (screws in a line, L4) and for ovine bones (screws in a square, S4) (AdhFix). Comparison of b) maximum load c) bending strength adjusted to the cross-section area of the fixation path and d) bending rigidity of the configurations using two layers of composite according to the present invention in porcine bones and ovine bones. Cyclic testing of composites on: e) porcine bones with L4 configuration and f) ovine bones with S4 configuration. Mean values shown with standard errors of mean (n=5).

### DETAILED DESCRIPTION OF THE INVENTION

Future standard-of-care injectable composite for fracture fixators are envisioned to be degradable upon proper bone regenerations. However, the rate of degradation should not jeopardize the mechanical properties that are necessary in bone fixation applications.

What the present inventors found was that by selecting specific monomers in a specific ratio and by selecting bioresorbable fillers a fully degradable composite, with a degradation rate slow enough such that its mechanical integrity is not compromised during bone healing can be made. The composite comprises a cured polymeric network and a ceramic component.

The present invention presents a novel injectable alternative to metal plates which combines the introduction of ester linkages in the organic phase and the mixture of HA with bioresorbable fillers. This composite platform amalgamates TATO derivatives with 1,3,5-triazine-2,4,6-trione, 1,3,5-tris(mercaptopropyl) (TMTATO, without ester linkages) and tris[(3-mercaptopropionyloxy)-ethyl]-isocyanurate (TEMPIC, with ester linkages) as thiol monomers and TATATO as the allyl monomer.

The present invention is based on the examples presented herein where six different combinations of TMTATO and TEMPIC have been tested having different molar ratio between TMTATO and TEMPIC: A: 100:0 (only TMTATO), B: 80:20, C: 60:40, D: 40:60, E: 20:80 and F: 0:100 (only TEMPIC). These polymeric matrixes were reinforced with bioresorbable fillers such as hydroxyapatite (HA), tricalcium phosphate (TCP) and bio glass (BG) (Figure 1).

Said examples show that combinations of TMTATO and TEMPIC as thiol monomers and HA/TCP or HA/BG as reinforcing filler exhibit the best balance of mechanical properties and degradation. All composites were investigated with respect to mechanical properties, water absorption, *in vitro* degradation and cytotoxicity towards keratinocytes (HaCaT) and mouse monocyte/macrophage-like cells (Raw 264.7). The most promising composites were evaluated as fracture fixators under 4-point bending with synthetic bones and under 3-point bending for monotonic failure and cyclic testing in porcine metacarpal and ovine phalanx bones with transverse fractures.

The mixture of TMTATO and TEMPIC should be selected so that the wanted properties of the obtained composite is obtained. The main properties affected by the ratio is mechanical properties and degradation time. A preferred TMTATO:TEMPIC molar ratio is 90-50:10-50, i.e. 90:10 to 50:50, within which the obtained composite material has high strength even during the degradation, and degrades within a suitable time period. At molar ratios of 80-60:20-40 said properties are even further pronounced. Both mechanical properties and water uptake is also influenced by the amount of said mixture. A preferred amount is therefore 25-45wt% of the total weight of the composite material, preferably 30-40wt%. TATATO is present in an amount of 15-30wt% of the total weight of the composite material, preferably 17-25wt%. The number of allyl groups and thiol groups may be stoichiometric or near stoichiometric to obtain a fully cured composite. As shown in the present application TEMPIC increased the degradation profile of the final composites. However, since the apparent softening behavior of TEMPIC affects the integrity of the composite as bone fracture fixator under physiological conditions the amount of TEMPIC must be adjusted according to the present invention. To high amount of TEMPIC resulted in softening of the composites below physiological temperatures which may result in failure when fixating bone for example.

Composites of the present invention comprises a filler where said filler is a ceramic component. The main purpose of the ceramic component of the composite is to provide additional strength. However, what the present inventors found was that by selecting specific components or fillers and by mixing specific ceramic components the degradation of the material may also be adjusted. As seen herein a strong composite material is provided with suitable degradation time. A mixture of hydroxyapatite and, bioglass or tricalcium phosphate results in surprising properties. An amount of the ceramic component of 30-60wt% based on the total weight of the composite material is preferred, preferably 40-55wt%, more preferably 45-53wt%. In order to further tailor the properties the amount of the different ceramic components may be altered. The hydroxyapatite and the bio glass and/or tricalcium phosphate of the ceramic component may be present in the composite material in an amount of 20-50wt% and 5-30wt% respectively such as 20-50wt% HA and, 5-30wt% of bioglass and/or tricalcium phosphate. Further improved properties are seen when the amount of hydroxyapatite is 30-45wt% and the amount of bio glass and/or tricalcium phosphate is 7-15wt%. What the present inventors have shown is that a mixture of hydroxyapatite and, bioglass or tricalcium phosphate, results in better mechanical properties even during degradation. Replacing hydroxyapatite with 10 and 20 wt% of tricalcium phosphate actually increased the flexural strength of the composites in comparison with pure hydroxyapatie, and replacing hydroxyapatite with bioglass also increased the flexural strength (Table 2).

In one embodiment of the second aspect of the present invention the composition comprises an initiator such as a photoinitiator selected from lithium phenyl(2,4,6-trimethylbenzoyl) phosphinate. Initiator may be present for example up to 5wt% of the composition such as 1-3wt%. By using a catalyst such as an amine catalyst the reaction rate increases. Unsaturated catalysts facilitate that the catalyst also reacts and becomes a part of the obtained composite material. A non-limiting amount of catalyst is 1-5wt%.

An advantage of the present invention is that it may be injectable and thereby facilitates that the composition may be applied in an easy and straight forward manner. It also makes it possible to apply the compositions to sites where premade composites are more difficult to arrange. In order for the composition to be injectable it should have a viscosity of 100 to 5,000psi.

Curing of the composition may be done using light with a spectral wavelength of e.g. 300-600nm. Time or number of pulses of light may be varied and depends on the thickness of the composite. As shown in the examples herein the heat exposed to the surrounding tissue during the curing does not significantly increase with increased exposure time when the composite thickness increases. Thereby the risk of tissue damage does not increase.

Flexural strength was shown to be dependent on the choice of ceramic component while the flexural modulus was dependent on the choice of ceramic component and the thiol mixture.

Water absorption and thereby degradation depends on the choice of thiol component and filler (ceramic component). What the present inventors found was that by the introduction of higher percentages of TEMPIC, the number of ester bonds increased, enhancing the water uptake of the TEMPIC rich thermosets.

### EXAMPLES

### Materials

All chemicals were purchased from commercial sources and used as received unless otherwise noted. 1,3,5-Triallyl-1,3,5-triazine-2,4,6(1H,3H,5H)-trione (TATATO) (98%), hydroxyapatite (HA, reagent grade, powder, synthetic), tricalcium phosphate (TCP, puriss. p.a., ≥98% β-phase basis), bioactive glass powder (BG, 58S, ≥98%, ≤10 µm particle size), and lithium phenyl(2,4,6-trimethylbenzoyl) phosphinate (TPO) (97%) were purchased from Sigma Aldrich Sweden AB. Thioacetic acid (98%) was purchased from Thermo Scientific. Hydrochloric acid (37%) and methanol (HPLC-gradient reagent) were purchased from VWR chemicals.Tris[(3-mercaptopropionyloxy)-ethyl]-Isocyanurate (TEMPIC) was purchased from BrunoBock. 1,3,5-Triazine-2,4,6-trione, 1,3,5-tris(mercaptopropyl) (TMTATO) was synthesized according to literature procedures.

### Instrumentation

Nuclear Magnetic Resonance (NMR): A Bruker 400 MHz NMR instrument was used to perform the experiments. The spectra were analyzed with MestReNova software. ¹H-NMR spectra were recorded at 400 MHz using 128 scans. ¹³C-NMR spectra were recorded at 101 MHz using 256 scans. CDCl3 solvent peak reference was set to 7.26 ppm for ¹H-NMR and 77.0 ppm (middle peak) for ¹³C-NMR spectra.

FT-Raman: A portable i-Raman Plus spectrometer (model: BWS465-785S, B&W TEK) was used to determine the complete conversion of thiols and alkenes before and after curing. The measurements were applied to A_HA₅₆/BG₃₈/TCP₂₈ or F_HA₅₆/BG₃₈/TCP₂₈ and B_HA_{42_}BG₁₀ and B_HA_{41_}TCP₁₀. A total amount of 32 scans (laser wavelength: 785 nm, laser power: 100%, integration time: 1000 ms) were used.

The raw data were analysed on BWSpec^{®} software and plotted in Origin 2020 (Academy). The carbonyl shift at 1760 cm⁻¹ was used to normalize the spectra. Thiol (2575 cm⁻¹) and alkene (1645 cm⁻¹) shifts were compared to confirm the full conversion of TEC reactions.

Scanning Electron Microscopy (SEM): A low-vacuum Hitachi Tabletop SEM TM-1000 (Japan), equipped with the BSE detector and a W filament was used. The voltage used was 15 kV and pictures were taken with a magnification of 50x. Samples were coated with a Cressington 208HR sputter coater with Pd target, using coating thicknesses between 3-5 nm to increase the conductivity of the samples.

Viscosity measurements: The viscosity evaluation of the uncured resins including A_HA₅₆/BG₃₈/TCP₂₈, F_HA₅₆/BG₃₈/TCP₂₈, B_HA_{42_}BG₁₀ and B_HA_{41_}TCP₁₀ were investigated using a DHR-2 rheometer (TA Instruments). A geometry gap of 300 µm was set and a parallel geometry of 20 mm of diameter was used. Flow sweep mode was selected to determine the relationship of the viscosity and the logarithm of the shear rate. Shear rate range was set between 10⁻⁴ and 10² 1/s (5 data points per decade) during the experiments. The raw data collected was analyzed with Trios software (TA Instruments). Three different samples were used per formulation and the results are shown as mean (Standard deviation, SD).

Instron: Four-point bending on PEEK bones, three-point bending on beams, configurations with composite and screws (AdhFix) on porcine and ovine bones and FRAPs on ovine bones and cyclic testing on AdhFix on porcine and ovine bones were evaluated with an Instron 5566 Universal Testing Machine.

Dynamic Mechanical Analysis (DMA): The glass transition temperatures (Tg) and onset points of the neat and all the polymer modified composites were measured by a Dynamic Mechanical Analyzer (DMA Q800, T.A. Instruments, USA) in thin film/tensile mode. The materials were of dimensions around 12 mm in length, 6 mm in width and 1.5 mm in thickness. The samples were tested under either dry or wet conditions. A temperature ramp method with a heating rate of 3 _{°}C/min was used and the testing temperatures between -10 _{°}C and 130 _{°}C. A strain of 0.1 % was induced with a frequency of 1 Hz. Five samples were tested for each formulation and time point.

### Methods

Formulation of composite materials: An unsaturated aliphatic amine catalyst (Cat57, 70 mg) and TATATO were mixed together with either TMTATO, TEMPIC or a mixture of both in a glass vial, always keeping a stoichiometric ratio between thiol and allyl groups. Then, the vial was protected from light and TPO (17.8 mg) was added. Finally, the maximum amount of HA, BG, TCP or a mixture of HA/BG or HA/TCP were added and mixed until a homogeneous and viscous mixture was obtained (Table 1).

**Table 1. Content of composite materials. All materials contained TATATO (1245.00 mg), Cat57 (70.00 mg) and TPO (17.80 mg).**

| **Formulation** | **Ratio¹** | **TMTATO (mg)** | **TEMPIC (mg)** | **HA (mg)** | **HA wt %** | **BG (mg)** | **BG wt %** | **TCP (mg)** | **TCP wt %** | **Potential degradation (%)** |
|---|---|---|---|---|---|---|---|---|---|---|
| **A_HA₅₆** | 100:0 | 1810.00 | - | 4000.0 | 56 | - | - | - | - | 0 |
| **A_BG₃₈** | 100:0 | 1810.00 | - | - | - | 1931.0 | 38 | - | - | 38 |
| **A_TCP₂₈** | 100:0 | 1810.00 | - | - | - | - | - | 1256.00 | 28 | 28 |
| **F_HA₅₆** | 0:100 | - | 2707.0 | 5141.0 | 56 | - | - | - | - | 29 |
| **F_BG₃₈** | 0:100 | - | 2707.0 | - | - | 2480.0 | 38 | - | - | 80 |
| **F_TCP₂₈** | 0:100 | - | 2707.0 | - | - | - | - | 1575.00 | 28 | 76 |
| **B_HA₅₆** | 80:20 | 1447.17 | 541.0 | 4226.0 | 56 | - | - | - | - | 7 |
| **C_HA₅₆** | 60:40 | 1086.38 | 1083.0 | 4457.0 | 56 | - | - | - | - | 14 |
| **D_HA₅₆** | 40:60 | 724.03 | 1624.0 | 4685.0 | 56 | - | - | - | - | 19 |
| **E_HA₅₆** | 20:80 | 362.13 | 2166.0 | 4913.0 | 56 | - | - | - | - | 25 |
| **B_BG₃₈** | 80:20 | 1447.17 | 541.0 | - | - | 2040.85 | 38 | - | - | 48 |
| **C_BG₃₈** | 60:40 | 1086.38 | 1083.0 | - | - | 2150.00 | 38 | - | - | 57 |
| **B_TCP₂₈** | 80:20 | 1447.17 | 541.0 | - | - | - | - | 1327.13 | 28 | 40 |
| **C_TCP₂₈** | 60:40 | 1085.88 | 1082.5 | - | - | - | - | 1370.00 | 28 | 50 |
| **B_HA_{42_}BG₁₀** | 80:20 | 1447.16 | 541.0 | 2866.7 | 42 | 687.89 | 10 | - | - | 18 |
| **B_HA_{26_}BG₂₀** | 80:20 | 1447.16 | 541.0 | 1623.2 | 26 | 1235.89 | 20 | - | - | 29 |
| **C_HA_{41_}BG₁₀** | 60:40 | 1086.38 | 1083.0 | 2866.2 | 41 | 707.50 | 10 | - | - | 25 |
| **C_HA_{28_}BG₂₀** | 60:40 | 1086.38 | 1083.0 | 1917.0 | 28 | 1355.00 | 20 | - | - | 36 |
| **B_HA_{41_}TCP₁₀** | 80:20 | 1447.16 | 541.0 | 2826.6 | 41 | - | - | 687.89 | 10 | 18 |
| **B_HA_{18_}TCP₂₀** | 80:20 | 1447.16 | 541.0 | 943.8 | 18 | - | - | 1066.48 | 20 | 30 |
| **C_HA_{40_}TCP₁₀** | 60:40 | 1086.38 | 1083.0 | 2846.3 | 40 | - | - | 704.08 | 10 | 25 |
| **C_HA_{17_}TCP₂₀** | 60:40 | 1086.38 | 1083.0 | 943.8 | 17 | - | - | 1111.29 | 20 | 40 |

| | | | | | | | | | | |
|---|---|---|---|---|---|---|---|---|---|---|
| ¹TMTATO:TEMPIC ratio | | | | | | | | | | |

Curing of composite materials: The resins were cured by a portable high-performance curing LED lamp (Bluephase 20i, Ivoclar Vivadent AG, Leichtenstein) with spectral wavelengths of 385-515 nm (dominant wavelengths of 400 and 470 nm) and light intensity of 2000 mW/cm². At least two pulses (5 seconds/pulse) of LED treatment were applied per cm² on both sides of the composite surface to ensure full conversion of monomers.

Three-point bending testing (beams): Mechanical analysis was conducted on rectangular samples of the cured composites, with dimensions 35 x 6 x 1.5 mm (length x width x thickness), immediately after curing (dry) and after being immersed in 50 mL (ratio 1/50 m/v) of phosphate buffered saline solution (PBS; pH= 7.4) at 37 °C for 1, 2, 4 or 8 weeks (wet). All samples were allowed to acclimatize to the testing temperature of 20 °C before testing began. The wet samples were then removed from their solutions and excess water on their surface was removed with tissue paper. Both dry and wet samples were tested on an Instron 5566 double column universal testing machine (Instron Korea LLC) with a load cell of 500 N and a crosshead speed of 1 mm/min, a preload of 0.1 N, and a preload speed of 0.5 mm/min. The center-to-center distance of the lower contacts was set to 30 mm and all the measurements were conducted at 20 °C with a relative humidity of 50%. The data were analysed and collected by Bluehill software. The flexural modulus was calculated by equation (1), where *L* is the lower contacts' distance, m is the slope at the initial elastic region of the load and displacement curve, w is the width of the beam, and d is the thickness of the beam. Five samples were tested for each formulation and time point. ${E}_{f} = \frac{{L}^{3} m}{4 {ωd}^{3}}$ Four-point bending: Mechanical analysis was conducted on synthetic bone substrates (cylindrical rods of PEEK with a diameter 10 mm) with ostectomies of 10 mm in length that were fixated with composite patches containing either the A_HA₅₆, B_HA_{42_}BG₁₀ and B_HA_{41_}TCP₁₀. The patches were anchored to the substrate with four bicortical screws (1.5 mm diameter) using a previously reported procedure known as AdhFix. The osteosyntheses were evaluated with four-point bending under dry conditions or after being soaked in PBS (pH7.4) at 37 °C for 1 week (wet conditions). All samples were allowed to acclimatize to the testing temperature of 20 °C before testing. The wet samples were then removed from their solutions and excess water on their surface was removed with tissue paper. Both dry and wet samples were tested on an Instron 5566 double column universal testing machine (Instron Korea LLC) with a load cell of 500 N, a crosshead speed of 1 mm/min, a preload of 0.5 N, and a preload speed of 0.5 mm/min. The support span was set to 45 mm and the loading and center span were set to 15 mm. All the measurements were conducted at 20 °C with a relative humidity of 50%. The data were analysed and collected by Bluehill software. Five samples were tested for each formulation and time point. Bending stiffness *K* was calculated at the initial elastic region of the load vs displacement curve, using equation 2, where F is the force and d is the displacement. The bending structural stiffness (EIₑ) was calculated with equation 3, where *h* is the loading span distance, *a* is the center span distance and K is the bending stiffness. Bending strength was calculated using equation 4, where P is the proof load and h is the loading span distance. The bending strength normalized with the cross-sectional area of the fixation patch was determined using equation 5, where *BS* is the bending strength, *w* is the width and *t* is the thickness of the fixation patch. $K = \frac{F}{d}$ ${EI}_{e} = \frac{\left(2h+3a\right) {Kh}^{2}}{12}$ $BS = \frac{Ph}{2}$ ${BS}_{CSA} = \frac{BS}{wt}$

Water absorption and degradation measurements: Water absorption and degradation tests were conducted on A_HA₅₆/BG₃₈/TCP₂₈, F_HA₅₆/BG₃₈/TCP₂₈, B_HA₅₆, B_BG₃₈, B_TCP₂₈ C_HA₅₆, C_BG₃₈, C_TCP₂₈, B_HA_{42_}BG₁₀, B_HA_{26_}BG₂₀, B_HA_{41_}TCP₁₀, B_HA_{18_}TCP₂₀, C_HA_{41_}BG₁₀, C_HA_{28_}BG₂₀, C_HA_{40_}TCP₁₀, and C_HA_{17_}TCP₂₀. Five disc-shaped samples were made in a plastic mold of diameter 12.5 mm and thickness 1.5 mm for each formulation and time point (1, 2, 4 and 8 weeks). Thereafter, samples were placed in a desiccator at 37 °C until the dried weight was constant within 0.1 mg (*m₂*)*.* Afterwards, they were stored in separated vials filled with 20 ml of PBS solution (pH= 7.4) to be completely submerged (ensuring a ratio at least 1/50 m/v), at 37 _{°}C. For water absorption, the mass of the samples was monitored at 1, 2, 4 and 8 weeks. The samples were taken out from the oven, washed with deionized water and the excess water was removed with tissue paper. The mass was then recorded (*m₂*) and they were returned to their corresponding vials and the oven until the last time point was reached. For degradation, at time points of either 1, 2, 4 or 8 weeks, the disks were removed from the PBS buffer and completely dried at 140°C until they obtained a constant dried weight (*m₃*)*.* Water absorption and degradation were calculated by using equations 6 and 7: $Water absorption \left(%\right) = \left|\frac{{m}_{2} - {m}_{1}}{{m}_{1}}\times 100\right|$ $Degradation \left(%\right) = \left|\frac{{m}_{3} - {m}_{1}}{{m}_{1}}\times 100\right|$

Three-point bending (bones): The mechanical performance of fixated transversal fractures on porcine and ovine bones were evaluated with a 10kN load cell. All measurements were conducted 20 C and 50% relative humidity. A center-to-center distance between the lower contacts of either 35 or 30 mm was used for the porcine metacarpal and ovine metatarsal, respectively. The maximum load and displacement were measured by using a cross-head speed of 5 mm min⁻¹, a preload of 1 N, and a preload speed of 2.5 mm min⁻¹. The bending rigidity was calculated from the initial elastic region of the slope of the load (F) versus displacement (Y) curve using equation 8, where L is the distance between the contacts. The fatigue testing of AdhFix on metacarpals was performed by subjecting the fixated bones to loads between 10 and 70 N continuously for 1000 cycles. The movement of the fixation was calculated as the difference between the maximum and minimum displacement values for the load cycle that caused the overall maximum displacement. Five specimens were tested for each configuration. All data were collected using Bluehill software: $Bending Rigidity = \frac{{L}^{2} ΔF}{48 ΔY}$

Cytotoxicity assays: A monolayer of human epidermal keratinocytes (HaCaT) and mouse monocyte cells (Raw 264.7) was used for the cytotoxicity tests. These cell lines were maintained in tissue culture flasks at 37 °C in 5 % CO₂ with Dulbecco's Modified Eagle Medium (DMEM), supplemented with 10% (v/v) Fetal Bovine Serum, 100 IU mL-1 penicillin and 100 µg mL⁻¹ streptomycin. A solution of 100 µL of the cells at a concentration of 5 × 10⁵ cells mL⁻¹ were harvested in 96-well plates for the cytotoxicity test and incubated for 24 h at 37 °C and 5 % CO₂. Meantime, disks made of neat composite, A_HA₅₆/BG₃₈/TCP₂₈, F_HA₅₆/BG₃₈/TCP₂₈, B_HA_{42_}BG₁₀ and B_HA₄₁_TCP₁₀ were exposed to UV light during 15 minutes. Afterwards, the solid material (discs of 12.5 mm of diameter and 1.5 mm of thickness) was transferred into 2 mL of complete DMEM and incubated at 37 °C for 24 hours, 1 week and 2 weeks to get the leach-out medium (the testing medium). Afterwards, old cell culture medium was replaced by 100 µL of the testing medium per well and incubated for 24 hours. For each sample medium, six parallel wells were used, and three discs were used for each material's formulation. Cells without treatment were used as a control and extracts without cells were used as blank of this experiment. Then, 10 µL of AlamarBlue agent was applied and incubated for 4 hours at 37 °C in 5 % CO₂. Finally, fluorescent intensity was measured with a plate reader (Tecan Infinite M200 Pro) at the wavelength of 560/590 nm (excitation/emission). Formulations showing less than 70% of cell viability were considered as toxic following ISO10993-5:200947 standard. ³⁹

### Results

By introducing chemical groups susceptible to hydrolysis, such as ester groups, while maintaining the TATO ring to keep rigidity, the interaction with water could be enhanced, increasing the water uptake and, thereby the degradation.

### Physiochemical properties of thiol-ene chemistry (TEC) formulations and composites

Interestingly, regardless the thiol derivative used, the maximum amounts of filler that could be added to the materials were found to be 56, 38 and 28 wt % for HA, BG or TCP, respectively (Table 1). Scanning electron microscopy (SEM) showed that the particle size of HA was significantly higher than TCP and BG with area values ranging from 636-4, 76-3 and 85-3 µm², respectively. This finding suggested that the maximum amount of fillers afforded was not driven by the particle size but by the compatibility and integration of the reinforcing filler with the polymeric matrix (Figure S2). The density of the fillers also varied, with TCP and BG having a significantly lower density than HA with 0.349, 0.441 and 1.427 g/cm³, respectively. (Figure S3). The higher amount of space occupied per mass of TCP and BG may have explained why less of these fillers could be incorporated into the formulation than HA. While each of the composite formulations were viscous fluids, rheological measurements showed that their viscosities differed depending on the filler and thiol monomer used. The viscosities of the TMTATO formulations (A) with HA, TCP and BG were 47.9, 65.0 and 44.7 % lower than their TEMPIC analogues (F). The choice of filler impacted the viscosity profile of the composites. Interestingly, the TCP and BG formulations showed drastic shear thinning behavior between shear rates of 10-⁴-10⁻¹ 1/s that continued until a shear rate of 10² 1/s, while the HA formulations showed a shear thickening behavior until a shear rate of 4×10⁻² 1/s, before changing to shear thinning until 10² 1/s shear rate (Figure S4).

The curing efficiency of the thermosets was determined by FT-Raman before and after exposure to high energy visual (HEV) light. In all formulations, regardless of the filler used, two 5 s pulses per cm² of HEV light were sufficient to consume all allyl and thiol groups for samples of 1.5 mm thick samples, proved by the complete disappearance of their peaks at 1645 and 2575 cm⁻¹, confirming the robustness of TEC curing composites including BG or TCP. As expected the TATO carbonyl signal at 1760 cm⁻¹ was unaffected by the curing reaction. The formulations containing TEMPIC also had a small peak at 1750 cm⁻¹ from the ester groups of the TEMPIC monomers. For thicker samples, more curing pulses might be needed, but the neglectable heat increase in the curing area, which increases from 20.8 to 22.5 °C after two HEV pulses, does not present any risk of soft tissue damage.

### Mechanical properties TEC composites.

Three-point bending showed that under dry conditions the flexural modulus of the composites was not affected by the choice of TMTATO or TEMPIC as the thiol component but was greatly dependent on the choice of HA, BG or TCP as filler (Figure 2a). The flexural moduli of the A_HA₅₆ and F_HA₅₆ composites were 6.8 (0.1) and 6.4 (0.3) GPa, respectively, which decreased to 5.5 (0.1) and 5.6 (0.1) GPa for the A_BG₃₈ and F_BG₃₈ composites and 4.2 (0.1) and 4.5 (0.1) GPa for the A_TCP₂₈ and F_TCP₂₈ composites, respectively. The decrease in modulus reflected the decrease in the maximum concentration of filler in the composites, from 56 to 38 to 28 wt% for HA to BG to TCP. On the other hand, the flexural strength of the composites was affected by both the choice of thiol and the choice of filler (Figure 2b). The A_HA₅₆ composite was stronger than the F_HA₅₆ composite, with flexural strength values of 64.5 (0.8) vs 58.1 (1.7) MPa. However, when HA was replaced with BG or TCP the flexural strength of the TMTATO composites decreased to 60.9 (2.0) and 50.7 (3.0) MPa, respectively, while the strength of the TEMPIC composites increased to 87.6 (3.4) and 73.8 (4.0) MPa, respectively. This suggested better integration of the BG and TCP fillers into the TEMPIC networks. All formulations under dry conditions showed onset points well above physiological temperature (37 °C; Figure 2 c); with the F_HA₅₆ formulation having the lowest onset point at 43.5 (0.8) °C.

The choice of thiol and filler both impacted the water absorption and degradation of the composites over 8 weeks submerged in PBS (pH= 7.4) at 37 °C (Figure 2d, e). The A_HA₅₆ composite absorbed the least water, with a mass increase of 1.72 (0.07) % after 8 weeks. Replacing HA with TCP or BG increased the water absorption to 3.45 (0.04) % and 4.25 (0.05), respectively. Replacing TMTATO with TEMPIC increased the water absorption of the HA, TCP and BG composites to 2.91 (0.03), 5.21 (0.14) and 5.65 (0.26) %, respectively. By the introduction of higher percentages of TEMPIC, the number of ester bonds increase, which enhances the water uptake of the TEMPIC rich thermosets by the interaction of the carbonyl group with the protons from the water. The mass loss of the composites due to hydrolytic degradation remained below 1 % for all formulations, except for the F_BG₃₈ composite, which increased from 1.79 (0.21) % after 2 weeks to 5.00 (0.17) and then 9.19 (0.12) % after 4 and 8 weeks.

Independent of filler type the TEMPIC increased the degradation profile of the final composites. However, the apparent softening behavior of TEMPIC jeopardizes integrity of the composite as bone fracture fixator under physiological conditions.

The composites were evaluated after submersion in PBS (pH= 7.4) at 37 °C for one week. The flexural modulus and strength, Tg and onset point were all found to be negatively correlated with the proportion of TEMPIC in these composites. Flexural modulus decreased from 5.0 (0.1) GPa for the formulation containing just TMTATO (A_HA₅₆) to 4.9 (0.2), 3.8 (0.2), 3.2 (0.2) and 1.7 (0.2) GPa for the composites B_HA₅₆, C_HA₅₆, D_HA₅₆ and E_HA₅₆, respectively (Figure S5a). Flexural strength followed the same pattern decreasing gradually from 43.8 (1.1) for A_HA₅₆ to 21.7 (2.6) for F_HA₅₆ (Figure S5b). The Tg of the different ratios decreased as expected, but it remained over 50 °C in all cases (Figure S5c); however, the onset point of the D_HA₅₆ and E_HA₅₆ composites also dropped below 45 °C, which significantly increases the risk of materials softening under physiological conditions (Figure S5d). These composite systems were therefore deemed inappropriate for use in fracture fixation and were excluded from further evaluation.

### Degradation

The replacement of HA with TCP or BG in the B_HA₅₆ and C_HA₅₆ composites decreased the flexural modulus, as previously seen in the A HA₅₆/BG₃₈/TCP₂₈ formulations. The extent of the decrease with TCP was similar for the B_TCP₂₈ and C_TCP₂₈ composites, with values of 4.3 (0.1) and 3.9 (0.1) GPa (a decrease of 32.2 and 38.4%) as it was with the A_TCP₂₈ composite (37.6%); however, with BG the decrease seen with the B_BG₃₈ and C_BG₃₈ composites was much lower than with the A_BG₃₈ composite, with values of 5.9 (0.1) and 5.8 (0.1) GPa (8.2 and 9.4%, respectively, compared to 18.5%; Figure 3a). Notably, including small amounts of TEMPIC in the formulation increased the modulus of the composites when BG was used as the filler. This effect was also seen in the flexural strength, where the C_BG₃₈ composite had the highest value of 73.7 (4.2) MPa (Figure 3b). When submerged in PBS (pH= 7.4) at 37 °C, the composites showed a gradual increase in water absorption over the whole 8-week period, with the exception of C_BG₃₈, reaching maximum water absorption after 2 weeks and then remained constant from 2-8 weeks (Figure 3d). The extent of water absorption was higher for the BG composites, followed by TCP, with the HA formulations absorbing the least water.

With regards to degradation, the HA formulations showed a slow and steady mass loss over time, with mass loss for B_HA₅₆ and C_HA₅₆ increasing from 0.28 (0.01) and 0.30 (0.01) % after one week to 0.38 (0.01) % and 0.45 (0.02) % after 8 weeks, respectively. The mass loss for the TCP-containing composites was steady from weeks 1-4 before increasing significantly between weeks 4 and 8, with the B_TCP₂₈ and C_TCP₂₈ composites having mass losses of 1.20 (0.09) and 1.07 (0.06) %, respectively, after 8 weeks. The mass loss of the BG-containing composites followed a different pattern. The C_BG₃₈ composite steadily loss mass throughout the 8-week period, eventually having a loss of 1.73 (0.02) % after 8 weeks. On the other hand, the B_BG₃₈ composite's mass loss actually decreased from weeks 2-4 (Figure 3e). This coincided with the formation of small crystals on the surface of the specimens after 4 weeks submerged in PBS (Figure S6). These crystals were likely due to remineralization of calcium phosphate from the PBS solution.

After being soaked for one week, all of the B and C formulations showed a decrease in flexural modulus. The softening of the materials due to the wetting process led to a decrease of 30.2, 53.9 and 59.2 % for the B_HA₅₆, B_BG₃₈ and B_TCP₂₈ composites, respectively, and of 39.8, 60.0 and 64.9 % for C_HA₅₆, C_BG₃₈ and C_TCP₂₈, respectively. There was no significant further change in modulus from weeks 1-8, with the exception of the BG formulations, where the modulus continued to decrease slightly until 2 weeks. The flexural strength of all the composites similarly decreased after week 1 but remained steady between weeks 1-8, with the exception of B_TCP₂₈ which remained steady over weeks 0-2, before decreasing slightly between weeks 4-8. The onset point was also affected after 8 weeks in wet conditions, lowering the values of C_HA₅₆, C_BG₃₈ and C_TCP₂₈ to 46.8 (0.2) 42.5 (1.7) and 42.0 (1.3) °C respectively, close to physiological temperature (Figure 3c). In contrast, the B ratio formulations all maintained onset points above 45 °C even after 8 weeks in PBS. However, the decrease in modulus and strength exhibited by the B_BG₃₈ and B_TCP₂₈ composites within 4 weeks of submersion in PBS may compromise their ability to stabilize a fracture in the crucial first month of healing, when the bone is remodeled by forming the hard callus. This suggested that HA could not be completely replaced with BG or TCP in the composite formulations.

In order to further optimize the composites such that degradation is maximized without sacrificing flexural modulus and strength, the B and C ratios were formulated with mixtures of HA and either TCP or BG. These mixtures included either 10 or 20 wt% TCP or BG, compensating with HA to keep the viscosity of the resins consistent with the previously described composites, which contained only one filler specie (Table S1). Increasing the ratio of TCP:HA decreased the flexural modulus under dry conditions, with the exception of B_HA_{41_}TCP₁₀, which actually had the highest modulus of all the B ratio formulations at 6.8 (0.1) GPa. As excepted, soaking the composites in PBS for 1 week decreased the modulus, with the formulations containing higher TCP content being more adversely affected. The modulus of the B_HA₄₁_TCP₁₀ and B_HA_{18_}TCP₂₀ composites was 3.2 (0.1) and 1.8 (0.1) GPa, respectively, after 1 week in PBS (Figure 4a). On the other hand, replacing HA with 10 and 20 wt% of TCP actually increased the flexural strength of the composites, especially those with the B ratio. For the C ratios, in dry conditions, the increase in flexural strength upon mixing TCP and HA was insignificant. However, when the composites were soaked in PBS the C_HA_{40_}TCP₁₀ and C_HA_{17_}TCP₂₀ formulations showed better strength values compared with the formulations containing either HA or TCP (Figure 4b).

The extent of water absorption and degradation increased with the concentration of TCP in both the B and C composite families (Figure 4c-d). After 1 week in PBS, the water absorption of the B_HA_{41_}TCP₁₀, B_HA_{18_}TCP₂₀ and B_TCP₂₈ composites was 40.6, 76.9 and 107.3 % higher, respectively, than the B_HA₅₆ composite. Likewise, the water absorption of the C_HA_{40_}TCP₁₀, C_HA_{17_}TCP₂₀ and C_TCP₂₈ were 21.7, 68.7 and 100.9 %, respectively, than the C_HA₅₆ composite (Figure 4c). All of the composites continued to absorb water throughout the 8-week submersion in PBS. After 8 weeks, the water absorption difference compared with B_HA₅₆ and C_HA₅₆ was similar, with increments of 64, 78 and 108 % for B_HA_{41_}TCP₁₀, B_HA_{18_}TCP₂₀ and B_TCP₂₈ respectively, and 14, 69 and 89 % C_HA_{40_}TCP₁₀, C_HA_{17_}TCP₂₀ and C_TCP₂₈, respectively. The degradation of the B_HA_{41_}TCP₁₀, B_HA_{18_}TCP₂₀ and B_TCP₂₈ composites was 47.5, 100.0 and 178.7 % higher, respectively than B_HA₅₆ after one week soaked in PBS. After 8 weeks in PBS, the B_HA₄₁_TCP₁₀ and B_HA_{18_}TCP₂₀ composites followed the same degradation pattern of B_HA₅₆, and B_TCP₂₈ slightly increased. For the C composites with TMTATO and TEMPIC in molar ratios of 60:40, the degradation profile from TCP composites did not show an increment over time when compared with the A_HA₅₆. The values compared with the A_HA₅₆ were 31.4, 98.1 and 176.2 % after one week and 22.9, 81.4 and 135.9 % after 8 weeks. All composites showed an onset point around 45 °C after 1 week submerged in PBS, with the exception of C_HA_{40_}TCP₁₀ which had an onset point of 38.8 (1.1) °C (Figure 4e). Further examination showed that the onset point of B_HA₄₁_TCP₁₀ remained constant after 1-8 weeks soaked in PBS (Figure 4f).

Similarly, the B and C systems where formulated with HA/BG. The HA:BG ratio used did not affect the flexural modulus under dry conditions for the composites containing 10 and 20 wt % BG; however, the B_BG₃₈ and C_BG₃₈ decreased by 8 and 9 %, respectively, with respect B_HA₅₆ and C_HA₅₆. Interestingly, increasing the BG content resulted in increased flexural strength, from 51.3 (1.7) MPa for B_HA₅₆ to 59.4 (2.0), 59.8 (1.2) and 61.6 (3.9) MPa for B_HA_{42_}BG₁₀, B_HA_{26_}BG₂₀ and B_BG₃₈, respectively. For the C ratio formulations, the flexural strength increased from 55.6 (1.0) MPa for C_HA₅₆ to 60.7 (2.0), 62.3 (1.8) and 73.7 (4.2) MPa for C_HA_{41_}BG₁₀, C_HA_{28_}BG₂₀ and C_BG₃₈. On the other hand, after submersion in PBS for 1 week the flexural modulus of the composites was found to decrease in relation to the increasing BG concentration (Figure 5a). Furthermore, flexural strength values decreased to between 35-45 MPa and remained constant up to 8 weeks in PBS (Figure 5b).

Water absorption increased steadily with increasing BG content, with higher values for the C composites compared to the B composites. (Figure 5c). After 8 weeks, the highest water absorption values were from the B_BG₃₈ composite, due to the values from the C_BG₃₈ composite being offset by its significant mass loss due to degradation. Surprisingly, the mass loss due to degradation of the B composites was not much affected by the HA:BG ratio; however, the values from the B_HA_{42_}BG₁₀ and B_HA_{26_}BG₂₀ composites at 4 and 8 weeks may be underestimated due to the presence of remineralized crystals (Figure 5d, Figure S6). These crystals were removed from the surface prior to weighing, but further crystals may have been entrapped within the samples. For the C composites, the extent of degradation did increase with the BG concentration. Unlike the HA:TCP composites, only one of the HA:BG composites, B_HA_{42_}BG₁₀, had an onset point above 45 °C after 1 week soaked in PBS. Further examination showed the onset point of B_HA_{42_}BG₁₀ was stable over the 8-week submersion in PBS (Figure 5e, f).

### Cytotoxic profile

Collectively, B_HA_{42_}BG₁₀ and B_HA_{41_}TCP₁₀ composite systems were considered the most promising based on their mechanical properties and slow degradation rates after 8 weeks period. Moreover, these composites were tested in HaCaT and Raw 264.7 cell lines showing excellent cell viability after 24 h proofing the absence of unreacted monomers leached out from the system.

### Mechanical performance as fracture fixators on synthetic bones

To further explore the mechanical performance of the composite as personalized and injectable bone fracture fixators, a 10 mm wide ostectomies in synthetic bones made of PEEK was used as adverse fracture model. An ostectomy fracture model was chosen to ensure that the synthetic bone substrate was not contributing to the stability of the fixation. The mechanical and degradation performance of B_HA_{42_}BG₁₀ and B_HA₄₁_TCP₁₀ were identified as the most promising with optimal ratio of TMTATO and TEMPIC and non-degradable HA and dissolvable TCP or BG in wet conditions. For comparison, the non-degradable A_HA₅₆ composite was also evaluated. In this context, the viscous formulations were injected, sculptured to anchored to the screws on each side of the synthetic bone fragments and finally cured with HEV-TEC to customized composite plates. The stabilized fracture models were evaluated with monotonic four-point bending under dry conditions and after 1 week soaked in PBS at 37°C. When loaded to failure, the fixations made with the three composites had similar bending stiffness and bending structural stiffness values. Under dry conditions, the bending strength of the degradable B_HA_{42_}BG₁₀ and B_HA_{41_}TCP₁₀ composites [210.9 (32.9) and 211.4 (23.0) Nmm, respectively] exceeded that of the non-degradable A_HA₅₆ composite [164.4 (12.1) Nmm] (Table 2). When these bending strengths were adjusted to the cross-section area of the fixation patches the difference increased even more. While the A_HA₅₆ showed a value of 11.6 (1.0) Nmm/mm², B_HA_{42_}BG₁₀ and B_HA₄₁_TCP₁₀ reached values of 20.9 (2.9) and 18.8 (1.0), outperforming the non-degradable composite formulation by 80 and 62% respectively.

**Table 2. Monotonic four-point bending results of fixation patches made with the non-degradable A_HA₅₆ and degradable B_HA_{42_}BG₁₀ and B_HA₄₁_TCP₁₀ composites on synthetic bone substrates with 10 mm ostectomies. Four screws were used to anchor the patches to the bone fragments. Mean values shown with standard errors of mean in parenthesis (n=5).**

| Formulation | Conditions | Bending Stiffness (N/mm) | Bending Structural Stiffness (N/m²) | Bending Strength (BS) (Nmm) | Bending Moment (Nmm) | BS adjusted for CSA¹ (Nmm/mm²) |
|---|---|---|---|---|---|---|
| A_HA₅₆ | Dry | 48.0 (5.3) | 0.068 (0.008) | 164.4 (12.1) | 204.7 (22.5) | 11.6 (1.0) |
| B_HA_{42_}BG₁₀ | Dry | 47.3 (6.2) | 0.066 (0.009) | 210.9 (32.9) | 244.1 (31.5) | 20.9 (2.9) |
| B_HA_{41_}TCP₁₀ | Dry | 43.0 (8.0) | 0.060 (0.011) | 211.4 (23.0) | 231.9 (31.4) | 18.8 (1.0) |
| A_HA₅₆ | Wet | 40.6 (5.4) | 0.057 (0.008) | 187.1 (35.5) | 304.7 (40.1) | 13.1 (1.1) |
| B_HA_{41_}TCP₁₀ | Wet | 45.7 (5.5) | 0.064 (0.008) | 123.7 (15.2) | 220.5 (33.4) | 9.2 (0.4) |
| B_HA_{42_}BG₁₀ | Wet | 42.8 (5.0) | 0.060 (0.007) | 174.7 (26.1) | 276.1 (23.4) | 10.5 (0.6) |

| | | | | | | |
|---|---|---|---|---|---|---|
| ¹ Cross section area of the patch | | | | | | |

Soaking the samples in PBS for 1 week did not have a significant impact on the properties of the non-degradable A_HA₅₆ composite; however, it did reduce the bending strength of the B_HA_{42_}BG₁₀ and B_HA₄₁_TCP₁₀ composites by 41 and 17%, respectively. When normalized by the CSA, their strengths decreased 57 and 44%, compared to their values under dry conditions. The similarities in strength and stiffness of the three composite patches was encouraging as previous studies have shown that fixations made with the non-degradable A_HA₅₆ composite have been strong enough to support fractures during rehabilitation exercises, exceeding the bending moment by a factor of at least 28, and *in vivo* bone healing.

### Mechanical performance as fracture fixators in porcine and ovine cadaver bones.

The B_HA_{42_}BG₁₀ and B_HA₄₁_TCP₁₀ composites were further exploited to fixate reduced transverse fractures in porcine metacarpal and ovine phalanx cadaver bones, with the non-degradable A_HA₅₆ composite being used as a benchmark system. Due to the different morphology of the porcine and ovine bones, different screw configurations were used to anchor the patches: four screws in a linear configuration for the porcine and four screws in a square configuration for the ovine bones (Figure 7a). The fixations were subjected to both monotonic and cyclic three-point bending. The maximum load achieved on the porcine bones under monotonic testing for the non-degradable A_HA₅₆ composite was 147.6 (13.4) N, while the B_HA_{42_}BG₁₀ and B_HA₄₁_TCP₁₀ composite achieved 136.7 (13.2) and 114.5 (4.2) N, respectively, before failure. When normalized by the CSA, the bending moments of the fixations on the porcine bones of the A_HA₅₆, B_HA_{42_}BG₁₀ and B_HA₄₁_TCP₁₀ composites were 137.1 (16.5), 116.1 (9.3) and 97.8 (5.4) Nmm/mm², respectively. Notably, the maximum loads collected on ovine bones for A_HA₅₆ and B_HA₄₁_TCP₁₀ were 530.7 (31.5) and 544.6 (35.5) N, respectively, with B_HA_{42_}BG₁₀ achieving a lower maximum load of 409.9 (43.0) N (Figure 7b). All three composites showed similar bending moments when normalized by CSA on the ovine bones of 226.4 (10.9), 207.5 (20.6) and 227.3 (15.78) Nmm/mm² for A_HA₅₆, B_HA_{42_}BG₁₀ and B_HA₄₁_TCP₁₀ respectively (Figure 7 c). The bending rigidity of the patches was similar regardless of bone substrate. For porcine bones, B_HA_{42_}BG₁₀ presented the highest value of 0.29 (0.03) Nm² followed by A_HA₅₆ with 0.25 (0.06) Nm² and B_HA₄₁_TCP₁₀ with 0.20 (0.02) Nm². On the ovine bones, A_HA₅₆ outperformed with 0.34 (0.02) Nm² followed by B_HA_{42_}BG₁₀ and B_HA₄₁_TCP₁₀ with similar values, 0.24 (0.04) and 0.25 (0.05) Nm², respectively (Figure 7d, Table 3). The mode of failure was different depending on the substrate, with the fixations on the porcine bones failing due to breakage of the patch over the fracture, while the patches on the ovine bones broke over the screw heads. From fixation point of view, it is obvious that the stabilization performance is not only dependent to mechanics of the composite but also the nature and quality of the bone. In this context, the ovine bone model is wider and more compact than porcine counterpart providing superior support and transferring the stress to the weakest area of the stabilizing system, the interface between the screwhead and the composite, of which the amount of composite is less than the rest of the patch.

**Table 3. Mechanical properties of AdhFix patches tested under monotonic 3-point bending. Mean values shown with standard errors of mean in parenthesis (n=5).**

| Samples | **Bone** | **Max Load (N)** | **Max. Compr. (mm)** | **Cross Section (mm²)** | **BM (Nmm)** | **BM CSA (Nmm/mm²)** | **BR (Nm²)** |
|---|---|---|---|---|---|---|---|
| **A_HA₅₆** | P | 142.2 (11.7) | 0.80 (0.15) | 9.5 (0.9) | 1244.4 (102.6) | 133.9 (13.7) | 0.28 (0.05) |
| **B_HA_{42_}BG₁₀** | P | 136.7 (13.2) | 0.60 (0.05) | 10.3 (0.6) | 1196.4 (115.2) | 116.1 (9.3) | 0.29 (0.02) |
| **B_HA_{41_}TCP₁₀** | P | 114.5 (4.2) | 0.59 (0.04) | 10.3 (0.3) | 1002.0 (37.0) | 97.8 (5.4) | 0.20 (0.02) |
| **A_HA₅₆** | O | 530.7 (31.5) | 1.45 (0.12) | 17.6 (0.4) | 3979.9 (236.4) | 226.4 (10.9) | 0.34 (0.02) |
| **B_HA_{42_}BG₁₀** | O | 409.9 (43.0) | 1.32 (0.25) | 14.9 (0.9) | 3074.4 (322.8) | 207.5 (20.6) | 0.24 (0.04) |
| **B_HA_{41_}TCP₁₀** | O | 544.6 (35.5) | 1.70 (0.29) | 18.0 (0.2) | 4084.2 (266.3) | 227.3 (15.8) | 0.25 (0.06) |

| | | | | | | | |
|---|---|---|---|---|---|---|---|
| BM = Bending moment, BR = Bending rigidity, P = Porcine, O =ovine | | | | | | | |

The stability of the fixations on porcine and ovine bones was also evaluated with cyclic flexural testing, using 1000 cycles of between 10 and 70 N, in order to simulate the flexing of fingers during hand fracture rehabilitation exercises. All fixations survived the 1000 cycles. On porcine bones, the B_HA_{42_}BG₁₀ patches were the most stable with a maximum displacement of 0.46 (0.02) mm and a movement of 0.137 (0.010) mm. The A_HA₅₆ patches were the least stable with a maximum displacement of 0.57 (0.09) mm and a movement of 0.132 (0.05) mm (Figure 7e). On ovine bones, all formulations showed similar stabilization with displacements of 0.41 (0.01), 0.41 (0.04) and 0.41 (0.07) for A_HA₅₆, B_HA_{42_}BG₁₀ and B_HA₄₁_TCP₁₀ respectively. However, the movement showed differences, A_HA₅₆ presented the shortest movement with 0.157 (0.021) mm while B_HA_{42_}BG₁₀ and B_HA₄₁_TCP₁₀ reached values of 0.172 (0.027) and 0.183 (0.021) mm respectively (Figure 7f, Table 4). This increased flexibility of the patches, together with the similar or lower values of displacement, indicates that degradable composites could be even more stable as fracture fixators as they could be more resistant to micro and macro motions.

**Table 4. Maximum displacement and movement values of porcine metacarpals and ovine metatarsal fixated fractures with AdhFix during 1000 cycles of 10 to 70 N in moist conditions. Mean values shown with standard errors of mean in parenthesis (n=5).**

| Formulation | Substrate | Max. Displacement (mm) | Movement (mm) |
|---|---|---|---|
| A_HA56 | Porcine | 0.57 (0.09) | 0.132 (0.005) |
| B_HA42_{_}B G10 | | 0.46 (0.02) | 0.137 (0.010) |
| B_HA41_TC P10 | | 0.55 (0.07) | 0.163 (0.010) |
| A_HA56 | Ovine | 0.36 (0.05) | 0.157 (0.021) |
| B_HA42_{_}B G10 | | 0.41 (0.04) | 0.172 (0.027) |
| B_HA41_TC P10 | | 0.41 (0.07) | 0.183 (0.021) |

### Mechanical performance as medical adhesive fixators of bone fracture.

The ability to fixate bone fractures without the use of metal implants that typically require harsh drilling conditions is one of the most desired solutions requested by the orthopedic community. Consequently, the B_HA_{42_}BG₁₀, B_HA_{41_}TCP₁₀ and A_HA₅₆ composites were explored as medical adhesives to fixate reduced transverse fractures in ovine bones using a bottom-up approach in which an adhesive primer was introduced as bone anchoring layer. The use of the primer, which etches the bone surface and intermolecularly crosslinks to the composites via thiol-ene chemistry, allows for metal-free bone fracture fixation without the need for drilling into the bone surface, called filler reinforced adhesive patch (FRAP). Interestingly, monotonic three-point bending revealed that the B_HA_{42_}BG₁₀ and B_HA₄₁_TCP₁₀ patches outperformed A_HA₅₆ in maximum load and extension before break. The degradable formulations achieved a maximum load of 163.7 (18.2) and 177.4 (20.7) N, respectively, while A_HA₅₆ achieved 129.3 (11.4) N Table 5. In all cases, failure was caused by adhesive detachment of the patch from the bone. A possible explanation to the higher maximum load of the degradable composites could have been due to their lower flexural modulus, which allows for more flexibility of the bone before failure. The higher rigidity of the A_HA₅₆ may have created more stress at the bone/primer interface, resulting in detachment at lower loads.

**Table 5. Mechanical properties of FRAPs on ovine bones with A_HA₅₆, B_HA_{42_}BG₁₀ and B_HA₄₁_TCP₁₀ composite formulations. Mean values shown with standard errors of mean in parenthesis (n=5).**

| Formulation | Maximum Load (N) | Patch Area (mm²) | BM (Nmm) | BM adjusted to AS (N/mm) | Load adjusted to AS (N/mm2) |
|---|---|---|---|---|---|
| A_HA₅₆ | 129.3 (11.4) | 142.2 (3.4) | 3878.4 (341.0) | 27.0 (2.0) | 0.9 (0.1) |
| B_HA_{42_}BG₁₀ | 163.7 (18.2) | 149.0 (3.1) | 4910.3 (546.4) | 32.9 (3.5) | 1.1 (0.1) |
| B_HA_{41_}TCP₁₀ | 177.4 (20.7) | 139.2 (4.3) | 5323.2 (619.7) | 39.0 (4.8) | 1.3 (0.2) |

| | | | | | |
|---|---|---|---|---|---|
| BM =Bending moment, AS= active surface | | | | | |

### Bendable and twistable thin film composites

To extend the usability of these composites beyond the scope of use as biomaterials for fracture fixations, thin films were also prepared. The viscous formulations of the A_HA₅₆, B_HA_{42_}BG₁₀ and B_HA₄₁_TCP₁₀ were casted and cured to films of 0.25 mm in thickness and 7.5 cm in length. All films were then after stored overnight in dry conditions at 37 °C, submerged in PBS at 37 °C and dry conditions at 70 °C, which was above their T_{g} of approximately 45 °C. Notably, all films under wet conditions presented a good bendability allowing for shape changing and twisting highlighting the softening effect of water thanks to its permeation and migration through the system, also reflected in the mechanical properties of the beams under wet conditions. However, in dry conditions there was a clear difference between A_HA₅₆ and the degradable counterparts.

At 37 °C, A_HA₅₆ broke with a distance between ends of 3.8 cm, while both degradable composite systems were able to bend until both edges touched each other. When the films were heated to 70 °C, the flexibilities increased for all three systems. For A_HA₅₆, the bendability increased and the films and broke at a distance of 3.0 cm between edges. In the case of degradable systems, the increased temperature enabled bending and twisting. The heat-promoted flexibility provided also more resistance against micro and macro motions that the patch could suffer during the healing process securing the fixations against undesired detachments. This was reflected in the maximum extension of the adhesive composite fixators withstanding increased extension of 67 and 59 % for B_HA_{42_}BG₁₀ and B_HA₄₁_TCP₁₀ respectively, before breakage when compared with A_HA₅₆.

Different composite mixtures have been formulated with different thiol monomers and ceramic components. Without being bound by theory but by including TEMPIC into the formulations, hydrolysable ester bonds have been integrated into the polymeric network, increasing the water absorption and introducing linkages susceptible to hydrolytic degradation. Resorbable ceramic components such as BG and TCP improved the water absorption and degradation compared with the A_HA₅₆ composite. Raman spectroscopy confirmed the efficiency of the TEC reaction regardless the thiol monomer and the inorganic filler used. While the complete replacement of TMTATO with TEMPIC and HA with BG or TCP did increase the water absorption and degradation, it was also accompanied by a significant decrease in flexural modulus and strength, especially under wet conditions. Composites B_HA_{42_}BG₁₀ and B_HA₄₁_TCP₁₀ comprising combined a mixture of TMTATO and TEMPIC in a 80:20 ratio and a mixture of HA and BG or TCP showed very promising results. Under wet conditions, their flexural modulus and strength was 4.1 (0.2) and 3.2 (0.1) GPa, and 45.0 (1.7) and 46.8 (1.2) MPa respectively. They maintained onset points of 52.3 (0.6) and 47.4 (0.6) °C even after 8 weeks soaked in PBS for B_HA_{42_}BG₁₀ and B_HA₄₁_TCP₁₀ respectively. Both formulations with a potential degradation of 18 wt%, showed a low degradation rate during the first week, improving the neat composite degradation by 27 and 42 % for B_HA_{42_}BG₁₀ and B_HA₄₁_TCP₁₀ respectively. When used for fixating ostectomies in synthetic bones, both B_HA_{42_}BG₁₀ and B_HA₄₁_TCP₁₀ provided stronger patches than the non-degradable A_HA₅₆ under dry conditions. Fixations were also made on reduced transverse fractures in porcine and ovine bones, using screws to anchor the fixation patch to the bone. Here, the degradable composites provided similar performance to the non-degradable A_HA₅₆ composite under monotonic testing and actually outperforming the non-degradable composite in terms of stability during cyclic testing up to loads of 70 N. The composites were also able to fixate ovine bones with reduced transverse fractures using an adhesive primer instead of screws for anchoring the patch to the bone. These metal-free fixations could withstand bending loads of up to 163.7 (18.2) and 177.4 (20.6) N before failure when made with the B_HA_{42_}BG₁₀ and B_HA₄₁_TCP₁₀ composites, respectively, which outperformed the 129.3 (11.4) N achieved with the non-degradable A_HA₅₆ composite. These properties combined with the good cytotoxicity profiles, demonstrated that simultaneously increasing the hydrolysable linkages in the polymer phase and the dissolvable content in the ceramic phase can result in composites combining high rigidity with slow degradation, which would be ideally suited for degradable bone fracture fixation implants.

## Claims

1. A composite material comprising a polymeric network and a ceramic component, wherein the polymeric network is obtained from 1,3,5-Triallyl-1,3,5-triazine-2,4,6(1H,3H,5H)-trione (TATATO) and a mixture of 1,3,5-triazine-2,4,6-trione, 1,3,5-tris(mercaptopropyl) (TMTATO) and tris[(3-mercaptopropionyloxy)-ethyl]-isocyanurate (TEMPIC), and wherein the ceramic component is a mixture of hydroxyapatite and, bio glass and/or tricalcium phosphate.

2. The composite material according to claim 1 wherein the mixture of TMTATO and TEMPIC has a molar ratio TMTATO:TEMPIC of 90-50:10-50, preferably 80-60:20-40.

3. The composite material according to claim 1 or 2 wherein the mixture of TMTATO and TEMPIC is present in an amount of 25-45wt% of the total weight of the composite material, preferably 30-40wt%.

4. The composite material according to any of the preceding claims wherein the TATATO is present in an amount of 15-30wt% of the total weight of the composite material, preferably 17-25wt%.

5. The composite material according to any of the preceding claims wherein the ceramic component is present in an amount of 30-60wt% of the total weight of the composite material, preferably 40-55wt%, more preferably 45-53wt%.

6. The composite material according to any of the preceding claims wherein the ceramic component comprises hydroxyapatite and, bio glass and/or tricalcium phosphate, and wherein hydroxyapatite and, bio glass and/or tricalcium phosphate is present in an amount of 20-50wt% and 5-30wt% respectively based on the total weight of the composition, preferably 30-45wt% and 7-15wt% respectively.

7. The composite according to any one of the preceding claims wherein the mixture of TMTATO and TEMPIC has a molar ratio TMTATO:TEMPIC of 90-50:10-50 and wherein the ceramic component comprises hydroxyapatite and, bio glass and/or tricalcium phosphate, and wherein hydroxyapatite and, bio glass and/or tricalcium phosphate is present in an amount of 20-50wt% and 5-30wt% respectively based on the total weight of the composition.

8. A composition comprising monomers, a ceramic component and a initiator, wherein the monomers are TATATO, and a mixture of 1,3,5-triazine-2,4,6-trione, 1,3,5-tris(mercaptopropyl) (TMTATO) and tris[(3-mercaptopropionyloxy)-ethyl]-isocyanurate (TEMPIC) in a 90-70:10-30 molar ratio, and wherein the ceramic component is a mixture of hydroxyapatite, and bio glass or tricalcium phosphate.

9. The composition according to claim 8 wherein the mixture of TMTATO and TEMPIC has a molar ratio of 90-50:10-50, preferably 80-60:20-40.

10. The composition according to claim 8 or 9 wherein the mixture of TMTATO and TEMPIC is present in an amount of 25-45wt% of the total weight of the composition, preferably 30-40wt%.

11. The composition according to any one of claim 8 to 10 wherein the TATATO is present in an amount of 15-30wt% of the total weight of the composition material, preferably 17-25wt%.

12. The composite material according to any one of claim 8 to 11 wherein the ceramic component is present in an amount of 30-60wt% of the total weight of the composition, preferably 40-55wt%, more preferably 45-53wt%.

13. The composition according to any one of claim 8 to 12 wherein the ceramic component comprises hydroxyapatite and bio glass and/or tricalcium phosphate, and wherein hydroxyapatite and bio glass and/or tricalcium phosphate is present in an amount of 20-50wt% and 5-30wt% respectively based on the total weight of the composition, preferably 30-45wt% and 7-15wt% respectively.

14. The composition according to any one of claim 8 to 13 wherein the initiator is selected from lithium phenyl(2,4,6-trimethylbenzoyl) phosphinate, lithium phenyl-2,4,6-trimethylbenzoylphosphinate, Ommirad 1000 (80% (2-hydroxy-2-methyl-1-phenylpropanone), 20% (1-hydroxycyclohexylphenyl ketone)), Omnirad 4265, Lucirin (main component ethyl(2,4,6-trimethylbenzoyl)-phenyl phosphinate) and Omnirad 4265 (50% Lucirin and 50% Omnirad 481 (1 (1-hydroxycyclohexylphenyl ketone)).

15. The composition according any one of claim 8 to 14 wherein the composition is injectable.
